# EUROPÄISCHE PATENTSCHRIFT

(11) **EP 3 129 324 B1**
(45) Veröffentlichungstag und Bekanntmachung des Hinweises auf die Patenterteilung: **16.09.2020**
(21) Anmeldenummer: 14815578.1
(22) Anmeldetag: 11.12.2014
(51) Int. Cl.: C01C 1/04

(54) **VERFAHREN ZUR HERSTELLUNG VON AMMONIAKGAS UND CO2 FÜR EINE HARNSTOFFSYNTHESE**
METHOD FOR PREPARATION OF AMMONIA GAS AND CO2 FOR A UREA SYNTHESIS PROCESS
PROCÉDÉ DE PRODUCTION DE GAZ D'AMMONIAC ET CO2 POUR UNE SYNTHESE DE L'UREE

(30) Priorität: 12.12.2013 DE 102013113980
(43) Veröffentlichungstag der Anmeldung: 15.02.2017
(73) Patentinhaber: thyssenkrupp AG, 45143 Essen (DE)
(72) Erfinder: MEISSNER, Christoph, 44135 Dortmund (DE); KROTOV, Denis, 44263 Dortmund (DE); VON MORSTEIN, Olaf, 45149 Essen (DE); KRÜGER, Matthias, Patrick, 44625 Herne (DE)
(74) Vertreter: thyssenkrupp Intellectual Property GmbH
(86) Internationale Anmeldenummer: PCT/EP2014/003315
(87) Internationale Veröffentlichungsnummer: WO 2015/086149

(56) Entgegenhaltungen:
- EP-A1- 1 031 534
- CN-A- 102 101 644
- DE-A1- 3 335 087
- JP-A- 2002 161 303
- KR-A- 20110 076 103
- US-A- 5 523 483

## Beschreibung

Die Erfindung betrifft ein Verfahren zur Herstellung von Ammoniak und CO₂ für eine Harnstoffsynthese.

Technisch wird Harnstoff aus NH₃ und CO₂ über die Zwischenstufe Ammoniumcarbamat gewonnen. Das Ammoniumcarbamat wird rasch und vollständig gebildet, wenn durch genügend hohen Reaktionsdruck eine Dissoziation vermieden wird. Das exotherm gebildete Ammoniumcarbamat wird in anschließenden Zersetzungsstufen bei niedrigem Druck endotherm in Harnstoff umgewandelt, wobei Überschussgase wieder in den Reaktor zurückgeführt werden können. Die Reaktion unter Bildung von Ammoniumcarbamat wird mit einem NH₃-Überschuss durchgeführt, wobei in der Praxis häufig ein Molverhältnis NH₃/CO₂ von etwa 4 gewählt wird.

Rohstoffe für die Harnstoffsynthese sind CO₂ und NH₃. Da Kohlendioxid als Nebenkomponente bei der Ammoniaksynthese anfällt, wird eine Harnstoffanlage häufig im Verbund mit einer Ammoniakanlage betrieben. Es werden Anlagen eingesetzt, die aus Erdgas, Luft und Wasser mit den Prozessschritten Wasserstoffherstellung, Ammoniakherstellung, Harnstoffsynthese schließlich Harnstoff produzieren.

Aus der JP 2002 161303A ist ein Verfahren zur Herstellung von Ammoniak und CO₂ aus Hochofengichtgas bekannt.

Aus der KR 2011 0076103 A ist ein Verfahren zur Ammoniak- und Harnstoffherstellung unter Verwendung von Konvertergas bekannt.

Vor diesem Hintergrund liegt der Erfindung die Aufgabe zugrunde, ein effizientes Verfahren zur Erzeugung der gasförmigen Edukte für die Harnstoffsynthese anzugeben. Zum Betreiben des Verfahrens soll ein Rohgas genutzt werden, welches als Abfallprodukt bei einem industriellen Prozess anfällt. Das Rohgas und die Verfahrensschritte sollen dabei so gewählt werden, dass die Gaskomponenten des Rohgases weitgehend vollständig und mit den für die Harnstoffsynthese notwendigen Anteilen in Ammoniak und CO₂ überführt werden.

Gegenstand der Erfindung und Lösung dieser Aufgabe ist ein Verfahren nach Anspruch 1. Vorteilhafte Ausgestaltungen des erfindungsgemäßen Verfahrens werden in den Patentansprüchen 2 bis 8 beschrieben.

Erfindungsgemäß wird zur Herstellung der gasförmigen Edukte für eine Harnstoffsynthese ein Hüttengas verwendet, welches aus einem Mischgas aus Hochofengichtgas und Konvertergas oder aus einem Mischgas aus Hochofengichtgas, Konvertergas und Koksofengas besteht. Hochofengichtgas fällt bei der Roheisenerzeugung in einem Hochofen an. Im Hochofen wird aus Eisenerzen, Zuschlägen sowie Koks und anderen Reduktionsmitteln wie Kohle, Öl oder Gas Roheisen gewonnen. Als Produkte der Reduktionsreaktionen entstehen zwangsläufig CO₂, Wasserstoff und Wasserdampf. Ein aus dem Hochofenprozess abgezogenes Hochofengichtgas weist neben den vorgenannten Bestandteilen einen hohen Gehalt an Stickstoff auf. Die Zusammensetzung des Hochofengichtgases ist abhängig von den Einsatzstoffen und der Betriebsweise und unterliegt Schwankungen. Üblicherweise enthält Hochofengichtgas jedoch 35 bis 60 Vol.-% N₂, 20 bis 30 Vol-% CO, 20 bis 30 Vol-% CO₂ und 2 bis 15 Vol.-% H₂:
Für das erfindungsgemäße Verfahren wird ein Hüttengas verwendet, welches aus einem Mischgas aus Hochofengichtgas und Konvertergas oder aus einem Mischgas aus Hochofengichtgas, Konvertergas und Koksofengas besteht. Konvertergas, welches im Konverterstahlwerk bei der Umwandlung von Roheisen zu Rohstahl anfällt, weist einen hohen Gehalt an CO auf und enthält ferner Stickstoff, Wasserstoff und CO₂. Eine typische Konvertergaszusammensetzung weist 50 bis 70 Vol.-% CO, 10 bis 20 Vol.-% N₂, ca. 15 Vol.-% CO₂ und ca. 2 Vol.-% H₂ auf. Koksofengas fällt bei der Verkokung von Kohle an und weist einen hohen Wasserstoffgehalt und beachtliche Mengen an CH₄ auf. Typischerweise enthält Koksofengas 55 bis 70 Vol.-% H₂, 20 bis 30 Vol.-% CH₄, 5 bis 10 Vol.-% N₂ und 5 bis 10 Vol.-% CO. Zusätzlich weist das Koksofengas Anteile von CO₂, NH₃ und H₂S auf.

Gemäß dem erfindungsgemäßen Verfahren wird aus dem Hüttengas ein Prozessgas erzeugt, welches Stickstoff, Wasserstoff und Kohlendioxid als Hauptkomponenten enthält und anschließend in einen den CO₂-Anteil enthaltenden Gasstrom und ein im Wesentlichen aus N₂ und H₂ bestehendes Gasgemisch aufgetrennt wird. Aus dem Gasgemisch wird durch Ammoniaksynthese ein für die Harnstoffsynthese geeignetes Ammoniakgas erzeugt. Aus dem CO₂-haltigen Gasstrom wird CO₂ in einer für die Harnstoffsynthese geeigneten Reinheit und Menge abgezweigt. Die Konditionierung des Hüttengases und die beschriebenen Trennschritte können so aufeinander abgestimmt werden, dass Ammoniak und CO₂ in den für die Harnstoffsynthese erforderlichen Anteilen gebildet werden und dass das Hüttengas annähernd vollständig zur Herstellung der für die Harnstoffsynthese benötigten gasförmigen Edukte genutzt werden kann.

Der Verwendung des Hüttengases zur Erzeugung von Prozessgas geht zweckmäßig eine Gasreinigung voraus. Die Gasreinigung dient der Abtrennung störender Inhaltstoffe, insbesondere von Teer, Schwefel und Schwefelverbindungen, aromatischen Kohlenwasserstoffen (BTX) und hochsiedenden Kohlenwasserstoffen.

Der CO-Anteil des Hüttengases kann durch eine Wasser-Gas-Shift-Reaktion in CO₂ und H₂ umgewandelt werden, wobei ein Prozessgas entsteht, welches als Hauptkomponenten Stickstoff, Wasserstoff und Kohlendioxid enthält.

Das Prozessgas wird anschließend vorzugsweise durch eine Druckwechseladsorption (PSA) in ein im Wesentlichen aus Stickstoff und Wasserstoff bestehendes Gasgemisch und ein den CO₂-Anteil enthaltendes Abgas - auch PSA-Offgas genannt - aufgetrennt. Eine im Stand der Technik bekannte Anwendung der Druckwechseladsorption (PSA-pressure swing adsorption) ist die Gewinnung und Reinigung von Wasserstoff. Im Rahmen des erfindungsgemäßen Verfahrens wird die Druckwechseladsorption in Kombination mit einer vorgeschalteten Gaskonditionierung so betrieben, dass sich ein gewünschtes Konzentrationsverhältnis von H₂ und N₂ einstellt. Ein Aspekt des erfindungsgemäßen Verfahrens ist daher die Kopplung einer Gaskonditionierung, insbesondere einer Wasser-Gas-Shift-Reaktion, mit einer Druckwechseladsorption, um aus Hüttengas, welches aus einem Mischgas aus Hochofengichtgas und Konvertergas oder aus einem Mischgas aus Hochofengichtgas, Konvertergas und Koksofengas besteht, ein für die Ammoniaksynthese geeignetes Synthesegas zu erzeugen. Ferner können durch die Druckwechseladsorption für die Ammoniaksynthese ungünstige Nebenkomponenten wie Argon, Methan oder Kohlenmonoxid entfernt oder in ihrer Konzentration verringert werden. Bei der Druckwechseladsorption fällt ein energiereiches Abgas (PSA-Offgas) an, welches den CO₂-Anteil des Prozessgases und etwaige Restanteile von CO enthält. Aus dem PSA-Offgas wird CO₂ für die Harnstoffsynthese gewonnen. Gemäß einer bevorzugten Ausführung des erfindungsgemäßen Verfahrens wird der CO₂-Anteil aus dem Abgas der Druckwechseladsorption (PSA-Offgas) abgetrennt und anschließend in ein CO₂ in hoher Konzentration enthaltendes Gas für die Harnstoffsynthese sowie ein Restgas mit einer geringeren CO₂- Konzentration aufgespalten.

Gegenstand der Erfindung ist auch ein Verfahren zur Herstellung von Harnstoff, bei dem aus Ammoniakgas und CO₂ unter Ammoniaküberschuss Ammoniumcarbamat erzeugt wird, welches in Wasser und Harnstoff gespalten wird. Erfindungsgemäß wird das für die Synthese von Harnstoff benötigte Ammoniakgas und das ebenfalls für die Synthese von Harnstoff benötigte CO₂ aus einem Hüttengas, welches aus einem Mischgas aus Hochofengichtgas und Konvertergas oder aus einem Mischgas aus Hochofengichtgas, Konvertergas und Koksofengas besteht, erzeugt. Wesentlich für das erfindungsgemäße Verfahren ist, dass die gasförmigen Edukte für die Harnstoffsynthese vollständig aus dem Hüttengas gewonnen werden. Die gasförmigen Edukte für die Harnstoffsynthese sind nach dem zuvor beschriebenen Verfahren erhältlich.

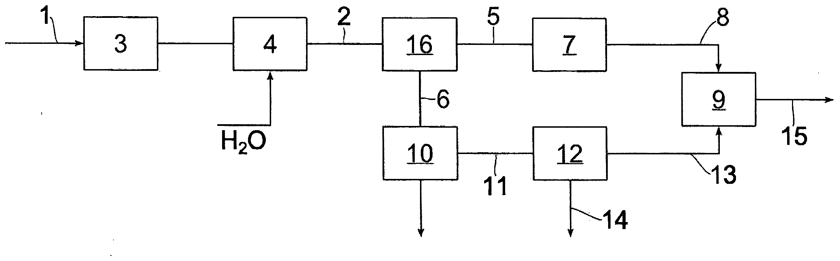

Im Folgenden wird die Erfindung anhand einer lediglich ein Ausführungsbeispiel darstellenden Zeichnung erläutert. Die einzige Figur zeigt schematisch in Form eines stark vereinfachten Blockschaltbilds ein Verfahren zur Herstellung von gasförmigen Edukten für eine Harnstoffsynthese.

Mit dem in der Figur dargestellten Verfahren wird aus einem Hüttengas 1, welches aus einem Mischgas aus Hochofengichtgas und Konvertergas oder aus einem Mischgas aus Hochofengichtgas, Konvertergas und Koksofengas besteht, ein Prozessgas 2 erzeugt, welches als Hauptkomponenten Stickstoff (N₂), Wasserstoff (H₂) und Kohlendioxid (CO₂) enthält.

Das Hochofengichtgas hat beispielsweise eine typische Zusammensetzung von 50 Vol.-% N₂) 24 Vol.-% CO₂, 21 Vol.-% CO und etwa 4 Vol.-% H₂. Nach einer Gasreinigung 3, in der störende Inhaltsstoffe, beispielsweise Teer, Schwefel und Schwefelverbindungen, aromatische Kohlenwasserstoff (BTX) und hochsiedende Kohlenwasserstoffe abgetrennt werden, wird das Hüttengas 1 mit einem Gasgemisch aus Hochofengichtgas und Konvertergas oder mit einem Gasgemisch aus Hochofengichtgas, Konvertergas und Koksofengas mittels einer Gaskonditionierung 4 in das Prozessgas 2 überführt, welches hauptsächlich aus N₂, H₂ und CO₂ besteht. Die Gaskonditionierung 4 beinhaltet insbesondere eine CO-Konvertierung, bei der der CO-Anteil des Hüttengases 1 durch eine Wasser-Gas-ShiftReaktion

CO + H₂O CO₂ + H₂

in CO₂ und H₂ umgewandelt wird. Nach der Konvertierung bzw. der Wasser-Gas-Shift-Reaktion hat das Prozessgas eine Zusammensetzung von ca. 37 Vol.-% CO₂, 21 Vol.-% H₂ und 42 Vol.-% N₂.

Das Prozessgas 2 wird durch eine Druckwechseladsoption (PSA) 16 in ein im Wesentlichen aus N₂ und H₂ bestehendes Gasgemisch 5 und ein den CO₂- Anteil enthaltendes Abgas 6 aufgetrennt. Aus dem N₂- und H₂-haltigen Gasgemisch wird durch eine Ammoniaksynthese 7 ein für die Synthese von Harnstoff geeignetes Ammoniakgas 8 erzeugt. Bei der Ammoniaksynthese 7 kann beispielsweise das Gasgemisch aus Wasserstoff und Stickstoff an einem Eisenoxidmischkatalysator bei Drücken zwischen 150 und 200 bar sowie bei einer Reaktionstemperatur von 350 bis 550 °C zur Reaktion gebracht werden.

Aus dem Abgas 6 der Druckwechseladsorption wird CO₂ für die Harnstoffsynthese 9 gewonnen. Mit dem in der Figur dargestellten Verfahrensschema wird in einer ersten Trennstufe 10 der CO₂-Anteil 11 aus dem Abgas 6 der Druckwechseladsorption abgetrennt. Anschließend erfolgt in einer zweiten Trennstufe 12 eine Aufspaltung in ein Kohlendioxid in hoher Konzentration enthaltendes Gas 13 und in ein Restgas 14 mit einer geringen CO₂ Konzentration. Bei dem Gas 13 handelt es sich insbesondere um Kohlendioxid in einer für die Harnstoffsynthese erforderlichen Reinheit.

CO₂ und NH₃ werden in den für die Harnstoffsynthese 9 erforderlichen Mengenanteilen der Harnstoffanlage zugeführt. In dieser wird unter Ammoniaküberschuss Ammoniumcarbamat erzeugt, welches in anschließenden Zersetzungsstufen bei niedrigem Druck in Harnstoff 5 umgewandelt wird.

Das anhand der Figur erläuterte Verfahren wird mit einem Gasgemisch aus Hochofengichtgas und Konvertergas oder mit einem Gasgemisch aus Hochofengichtgas, Konvertergas und Koksofengas als Hüttengas 1 betrieben.

## Patentansprüche

1. Verfahren zur Herstellung von Ammoniakgas und CO₂ für eine Harnstoffsynthese,
wobei aus einem Hüttengas (1), welches aus einem Mischgas aus Hochofengichtgas und Konvertergas oder aus einem Mischgas aus Hochofengichtgas, Konvertergas und Koksofengas besteht, ein Prozessgas (2) erzeugt wird, welches Stickstoff, Wasserstoff und Kohlendioxid als Hauptkomponenten enthält,
wobei das Prozessgas (2) in einen den CO₂-Anteil enthaltenden Gasstrom(6) sowie ein im Wesentlichen aus N₂ und H₂ bestehendes Gasgemisch (5) aufgetrennt wird,
wobei aus dem Gasgemisch (5) durch Ammoniaksynthese (7) ein für die Harnstoffsynthese (9) geeignetes Ammoniakgas (8) erzeugt wird und
wobei aus dem CO₂-haltigen Gasstrom (6) CO₂ in einer für die Harnstoffsynthese(9) geeigneten Reinheit und Menge abgezweigt wird.

2. Verfahren nach Anspruch 1, **dadurch gekennzeichnet, dass** das Hüttengas (1) vor seiner Verwendung zur Erzeugung von Prozessgas gereinigt wird.

3. Verfahren nach einem der Ansprüche 1 bis 2, **dadurch gekennzeichnet, dass** der CO-Anteil des Hüttengases (1) durch eine Wasser-Gas-Shift-Reaktion (4) in CO₂ und H₂ umgewandelt wird.

4. Verfahren nach einem der Ansprüche 1 bis 3, **dadurch gekennzeichnet, dass** das Prozessgas (2) durch eine Druckwechseladsorption (16) in ein im Wesentlichen aus N₂ und H₂ bestehendes Gasgemisch (5) und ein den CO₂-Anteil enthaltendes Abgas (6) der Druckwechseladsorption aufgetrennt wird.

5. Verfahren nach Anspruch 3 oder 4, **dadurch gekennzeichnet, dass** die Druckwechseladsorption (16) so betrieben wird, dass das Prozessgas (2) N₂ und H₂ in einem für die Ammoniaksynthese (7) geeigneten Konzentrationsverhältnis enthält.

6. Verfahren nach Anspruch 4 oder 5, **dadurch gekennzeichnet, dass** aus dem Abgas (6) der Druckwechseladsorption (16) CO₂ für die Harnstoffsynthese (9) gewonnen wird.

7. Verfahren nach Anspruch 6, **dadurch gekennzeichnet, dass** der CO₂-Anteil (11) aus dem Abgas (6) der Druckwechseladsorption abgetrennt und anschließend in ein Kohlendioxid in hoher Konzentration enthaltendes Gas (13) für die Harnstoffsynthese (9) und ein Restgas (14) mit einer geringeren CO₂- Konzentration aufgespalten wird.

8. Verfahren zur Herstellung von Harnstoff, wobei aus Ammoniakgas und CO₂ unter Ammoniaküberschuss Ammoniumcarbamat erzeugt wird, welches in anschließenden Zersetzungsstufen bei niedrigem Druck in Harnstoff umgewandelt wird, **dadurch gekennzeichnet, dass** das für die Synthese von Harnstoff benötigte Ammoniakgas und das ebenfalls für die Synthese von Harnstoff benötigte CO₂ aus einem Hüttengas, welches aus einem Mischgas aus Hochofengichtgas und Konvertergas oder aus einem Mischgas aus Hochofengichtgas, Konvertergas und Koksofengas besteht, erzeugt wird.

## Claims

1. Process for preparing ammonia gas and CO₂ for a urea synthesis,
wherein a process gas (2) containing nitrogen, hydrogen and carbon dioxide as main components is produced from a metallurgical gas (1) which consists of a mixed gas composed of blast furnace gas and converter gas or of a mixed gas composed of blast furnace gas, converter gas and coke oven gas,
wherein the process gas (2) is split to give a gas stream (6) containing the CO₂ proportion and a gas mixture (5) consisting essentially of N₂ and H₂,
wherein an ammonia gas (8) suitable for the urea synthesis (9) is produced from the gas mixture (5) by means of ammonia synthesis (7) and
wherein CO₂ is branched off from the CO₂-containing gas stream (6) in a purity and amount suitable for the urea synthesis (9).

2. Process according to Claim 1, **characterized in that** the metallurgical gas (1) is purified before being used for producing process gas.

3. Process according to any of Claims 1 to 2, **characterized in that** the CO proportion of the metallurgical gas (1) is converted into CO₂ and H₂ by means of a water gas shift reaction (4).

4. Process according to any of Claims 1 to 3, **characterized in that** the process gas (2) is split by means of pressure swing adsorption (16) to give a gas mixture (5) consisting essentially of N₂ and H₂ and an offgas (6) containing the CO₂ proportion from the pressure swing adsorption.

5. Process according to Claim 3 or 4, **characterized in that** the pressure swing adsorption (16) is operated in such a way that the process gas (2) contains N₂ and H₂ in a concentration ratio suitable for the ammonia synthesis (7) .

6. Process according to Claim 4 or 5, **characterized in that** CO₂ for the urea synthesis (9) is obtained from the offgas (6) from the pressure swing adsorption (16).

7. Process according to Claim 6, **characterized in that** the CO₂ proportion (11) is separated off from the offgas (6) from the pressure swing adsorption and is subsequently split up into a gas (13) containing a high concentration of carbon dioxide for the urea synthesis (9) and a tailgas (14) having a lower concentration of CO₂.

8. Process for preparing urea, where ammonium carbamate is produced from ammonia gas and CO₂ using an excess of ammonia and this ammonium carbamate is converted into urea in subsequent decomposition stages at low pressure, **characterized in that** the ammonia gas required for the synthesis of urea and the CO₂ which is likewise required for the synthesis of urea are produced from a metallurgical gas which consists of a mixed gas composed of blast furnace gas and converter gas or of a mixed gas composed of blast furnace gas, converter gas and coke oven gas.

## Revendications

1. Procédé pour la préparation de gaz d'ammoniac et de CO₂ pour une synthèse d'urée, un gaz de procédé (2) étant produit à partir d'un gaz métallurgique (1) qui est constitué d'un mélange de gaz composé de gaz de hauts fourneaux et de gaz de convertisseur ou d'un mélange de gaz composé de gaz de hauts fourneaux, de gaz de convertisseur et de gaz de cokerie, lequel gaz de procédé contient de l'azote, de l'hydrogène et du dioxyde de carbone en tant que composants principaux,
le gaz de procédé (2) étant séparé en un flux de gaz (6) contenant la partie de CO₂ ainsi qu'en un mélange de gaz (5) constitué essentiellement de N₂ et de H₂, à partir du mélange de gaz (5), par synthèse d'ammoniaque (7), un gaz d'ammoniaque (8) approprié pour la synthèse d'urée (9) étant produit et
à partir du flux de gaz (6) contenant du CO₂, du CO₂ en une pureté et une quantité appropriées pour la synthèse d'urée (9) étant dévié.

2. Procédé selon la revendication 1, **caractérisé en ce que** le gaz métallurgique (1) est purifié avant son utilisation pour la production de gaz de procédé.

3. Procédé selon l'une quelconque des revendications 1 et 2, **caractérisé en ce que** la partie de CO du gaz métallurgique (1) est convertie par une réaction de déplacement de gaz à l'eau (4) en CO₂ et H₂.

4. Procédé selon l'une quelconque des revendications 1 à 3, **caractérisé en ce que** le gaz de procédé (2) est séparé par l'intermédiaire d'une adsorption modulée en pression (16) en un mélange de gaz (5) essentiellement constitué de N₂ et H₂ et en un gaz d'échappement (6) contenant la partie de CO₂ de l'adsorption modulée en pression.

5. Procédé selon la revendication 3 ou 4, **caractérisé en ce que** l'adsorption modulée en pression (16) fonctionne de telle façon que le gaz de procédé (2) contient du N₂ et du H₂ en un rapport de concentration approprié pour la synthèse d'ammoniac (7) .

6. Procédé selon la revendication 4 ou 5, **caractérisé en ce qu'**à partir du gaz échappement (6) de l'adsorption modulée en pression (16), du CO₂ pour la synthèse d'urée (9) est obtenu.

7. Procédé selon la revendication 6, **caractérisé en ce que** la partie de CO₂ (11) du gaz échappement (6) de l'adsorption modulée en pression est séparée et ensuite est scindée en un gaz (13) contenant du dioxyde de carbone en concentration élevée pour la synthèse d'urée (9) et en un gaz résiduel (14) comportant une concentration en CO₂ plus faible.

8. Procédé pour la préparation d'urée, du carbamate d'ammonium étant produit à partir de gaz d'ammoniaque et de CO₂ avec un excès d'ammoniac, lequel est converti en urée dans des étapes de décomposition ultérieures sous pression réduite, **caractérisé en ce que** le gaz d'ammoniac nécessaire pour la synthèse d'urée et le CO₂ également nécessaire pour la synthèse d'urée sont produits à partir d'un gaz métallurgique qui est constitué d'un mélange de gaz composé de gaz de hauts fourneaux et de gaz de convertisseur ou d'un mélange de gaz composé de gaz de hauts fourneaux, de gaz de convertisseur et de gaz de cokerie.
